(19) Europäisches Patentamt / European Patent Office / Office européen des brevets

(11) **EP 3 658 892 B1**

(12) **EUROPEAN PATENT SPECIFICATION**

(45) Date of publication and mention
of the grant of the patent:
**14.07.2021 Bulletin 2021/28**

(21) Application number: **18746120.7**

(22) Date of filing: **18.07.2018**

(51) Int Cl.:
*G01N 17/00* (2006.01)    *B01L 1/02* (2006.01)

(86) International application number:
**PCT/EP2018/069501**

(87) International publication number:
**WO 2019/020455 (31.01.2019 Gazette 2019/05)**

(54) **A CITY POLLUTION ENVIRONMENT SIMULATION APPARATUS**

VORRICHTUNG ZUR SIMULATION VON STÄDTISCHER UMWELTVERSCHMUTZUNG

APPAREIL DE SIMULATION D'ENVIRONNEMENT DE POLLUTION URBAINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.07.2017 PCT/CN2017/094865**

(43) Date of publication of application:
**03.06.2020 Bulletin 2020/23**

(73) Proprietor: **BASF SE**
**67056 Ludwigshafen am Rhein (DE)**

(72) Inventors:
• **RAO, Zhi**
  **Shanghai 200137 (CN)**
• **STADLER, Daniel**
  **67056 Ludwigshafen (DE)**
• **CHEN, Rodney**
  **South Yarra, VIC 3141 (AU)**

(56) References cited:
**CN-A- 101 887 042      DE-B3-102005 047 326**
**KR-B1- 101 737 611**

## Description

### Technical Field

The present invention relates to an apparatus for testing environmental pollution effects and efficacy of personal care compositions for cosmetic or pharmaceutical use against such effects. Particularly, the invention relates to an apparatus for testing anti-pollution efficacy of personal care compositions by simulating the city atmospheric environment, and investigating damages caused by urban pollution on hair, skin and/or other substrates before and after applying such compositions. Hence, the effects of such compositions or their individual ingredients on hair and/or skin, or the direct impact of urban air pollution on compounds as such. More particularly, the invention relates to an experiment equipment for simulating the atmospheric environment in the city in laboratories and methods for operating said equipment for the aforementioned purpose.

### Background Art

Fast urbanization in metropolitan areas and the rise of megacities worldwide lead to a grow of city pollution, affecting the health and thereby also the appearance of inhabitants of such areas. The cosmetic and pharmaceutical industry is confronted with the demand of consumers of providing personal care compositions addressing this issue, and protecting from and preventing the consequences of city pollution effects on health, hair and skin. The term "city pollution", as used herein, encompasses acknowledged sources of environmental origin having harmful effects on skin and hair such as air pollution and UV radiation. Air pollution occurs when harmful substances, including particulates and molecules, are present in the air, and UV radiation constitutes about 10% of the total light output of the sun. The latter may also be produced by electric arcs and specialized lights, such as e.g. tanning lamps in solariums. Long-wavelength ultraviolet radiation can cause chemical reactions and consequently, the biological - and potentially damaging and harmful - effects of UV are greater than simple heating effects, and may derive from its interactions with organic molecules.

In the market, a lot of personal care products are sold with "anti-pollution" claims, but the scientific proof for these claims have been difficult to mimic and have had in the past poor relevance with regard to the real city pollution environment. Although UV damages on the human body are well known for many years, the effects in combination with other environmental stress factors on health, hair and skin have been less evaluated.

Especially as known environmental factors that affect skin include not only UV radiation (UVR), but as well major air pollutants such as polycyclic aromatic hydrocarbons (PAHs), volatile organic compounds (VOCs), nitrogen oxide (NOx) and particulate matter (PM). Air pollutants may interfere with the normal functioning of lipids, DNA and proteins in the human skin via oxidative damage, leading to skin cancer, skin ageing and inflammatory or allergic conditions such as urticaria, eczema, atopic dermatitis, psoriasis and acne. One of the main pollutants which affect the skin is for instance, ozone, which oxidizes sebum and causes damages the cutaneous barrier and skin inflammation. Fine particles act as well harmfully, especially those of a particle size of PM2.5, which cling to the skin and become lodged in the pores, possibly penetrating down to the epidermis. When pollutants are combined with UV rays, they generate a negative synergy, hence the accumulated damage from pollutants combined with UV rays is stronger than the damage caused from two of them separate. This has been pointed out in several publications disclosing the effects being amplified when multiple factors work in conjunction, such as UVR (UV radiation) interacting with air pollution or the presence of several air pollutants at the same time. These combinations are major active components of pro-oxidants smog (Hui Y. et al, (2015). Climate Change and Its Dermatologic Impact on Aging Skin. In Textbook of Aging Skin, Springer Ed., pp 1-8).

Methods are still under development to better understand, evaluate and - in consequence thereof - fight against the effects of major pollutants such as particulate matters, VOCs or ozone concentration.

Thus, there is still especially the need to establish methods, which can simulate the combined effects of UVR and air pollution.

Up to now, in the personal care industry, the investigation of pollution effect on hair, health and skin was done in a number of ways which were not highly relevant to real city pollution environment. Furthermore, in order to evaluate environmental conditions, and especially air pollution effects, either on personal care products as such, or on human substrates, such as hair and/or skin or on human derived substances, like sebum or squalene, only a few possibilities of doing so have been disclosed up to now.

For instance, although effects of anti-pollution effective compounds and compositions have been discussed in literature, the technical details and technological background of the so-called" urban pollution test model", wherein pollution effects were caused by simultaneous exposure to exhaust gas and UV rays on hair, was not disclosed (EURO COSMETIC, Vol. 11, 4-2003, p.19).

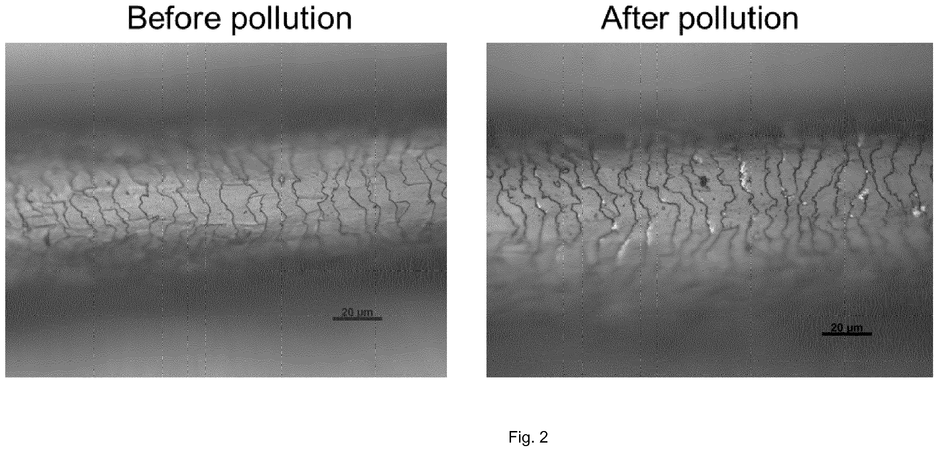

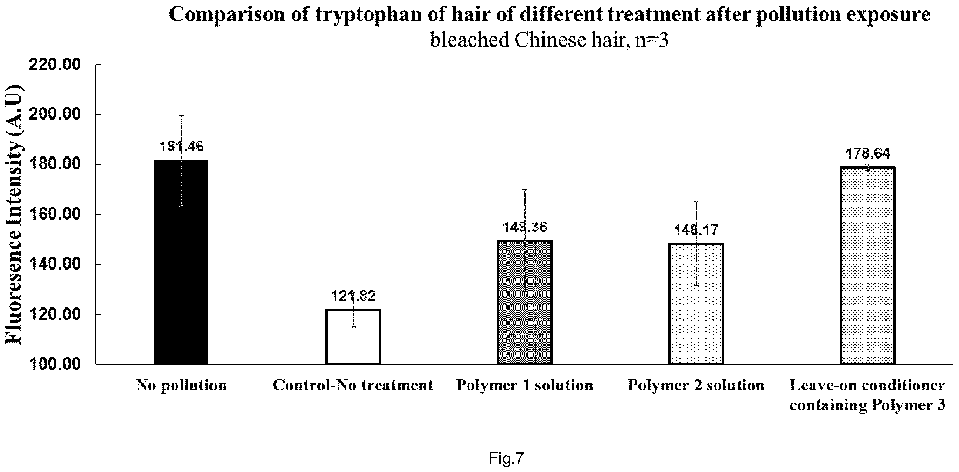

Likewise, in other publications, the effects of cigarette smoke and long UVA rays on squalene, a compound produced by the human sebaceous gland, were tested in a laboratory scale (Int J Cosmetic Science, 2015, 37, 357-365, Fig 7). However, cigarette smoke cannot be regarded as substituting for atmospheric air pollution. Same argumentation applies also to another pollution model, the "sebum pollution model", which refer to pollution as "atmospheric particulate matter combined with grease/oils typical of human sebum" (Intl Journal of Cosmetic Science, 2016, 1-4, Fig.2). The test model compares the efficacy of a sonic cleansing brush to manual cleansing. Also, this pollution model does not reflect air pollution effects on individual human derived samples, the objective of the present invention.

CN 101 887 042 A describes a multifunctional environment simulation test chamber for an on-line detecting textile material total organic volatile, comprising a main chamber body, an ultraviolet lamp, an ultrasonic humidifier, a VOC (Volatile Organic Compounds) sensor, an electric heater, a refrigerating compressor and a circulating fan. The main chamber body is provided with a sample injecting switch at the top end, an airtight door at the front surface, a cleaning drainage valve and an exhaust sampling electromagnetic valve at the bottom and the ultraviolet lamp inside; the ultrasonic humidifier is arranged on one side of the airtight door, the VOC sensor is arranged on the side surface of the main chamber body, the electric heater is connected with the main chamber body by a circulating air pipe, the refrigerating compressor is arranged on the lower part of the main chamber body, and the circulating fan is arranged below the electric heater.

KR 101 737 611 B1 describes a system to analyze fine dust blocking ability of cosmetics using an apparatus to form an artificial atmospheric environment including fine dust. An artificial atmospheric environment formation apparatus (1) of KR 101 737 611 B1 comprises: a chamber (10) to form fine dust; a wind direction generation device (20) including a plurality of fans (21, 22, 23, 24) arranged inside the chamber (10) at different angles to fluidize a supplied dust in the chamber (10) in order to distribute the supplied dust in a manner identical or similar to an actual atmospheric environment; and a dust supply unit (30) integrally provided with the chamber or separately provided from the chamber (10) to supply the fine dust into the chamber (10). The system of KR 101 737 611 B1 comprises: a capturing device (100) to capture an image of an object divided into a coated part coated with cosmetics, and a non-coated part not coated with the cosmetics before and after exposure to the fine dust; the artificial atmospheric environment formation device apparatus (1) exposing the object to the fine dust; and an image analysis device (200) to compare and analyze the images capturing the object before and after exposure to the fine dust.

The simulation of environmental conditions combing different air pollution factors, such as humidity, exhaust gas and/or UV irradiation, and allowing the evaluation of single factor as well as multi factors pollution environment in order to mimic the atmospheric condition have been disclosed in CN 204583213U and corresponding CN 104707671A. Still both publications describe an environmental atmosphere simulation walk-in equipment mimicking the atmospheric condition of the environment, allowing the operators to walk-in and arrange the test setting inside the environmental simulation chamber. The equipment disclosed therein is divided into an external structure and an internal structure, wherein the external structure includes a cabin body, a hatch door, an integrated control rack, an artificial simulation pollutant source, a gaseous processing system and a gas gathering system, and wherein the internal structure includes a stainless steel meshwork, a laboratory bench, a gas circulation system, an atmospheric control system, an atmosphere and particles pollutant sampling sensor and ultraviolet visible lights.

However, although the experimental equipment of CN 204583213U and CN 104707671A is described for simulating different environmental conditions, and includes various air pollution factors, it is not suitable to match the needs of a conventional laboratory standard apparatus for evaluating small samples in a simple and easy-to-handle manner, without the operator having to enter the apparatus. Especially the latter would require special precautious measurements for avoiding exposure of the operator to the pollution environment.

Hence, for the research and development of new anti-pollution products for personal care on a laboratory scale, there is still the need for a laboratory apparatus, which fits in a conventional fume hood, is easy to handle, easy to clean and maintain, and can be operated by one single person and which can be operated from the outside. In order to save resources, the objective is further to obtain the evaluation results within a limited period of time, within the limited space of a conventional laboratory fume hood, with a minimal manpower for operation and without the need of special safety measurements and protection equipment for the operator.

## Summary of the Invention

The object of the present invention is to provide a laboratory apparatus fitting into a conventional laboratory fume hood for simulating the city pollution environment in order to evaluate the antipollution efficacy of personal care compositions and products on hair, scalp and/or skin, as well as on other substrates or the compositions and active compounds as such, which is easy to operate and clean, and does not require special safety precautions for the operator.

The object of the present invention is further to provide a method to evaluate the pollution effects on hair and/or skin (as well as other substrates) by simulating the city pollution environment.

To achieve these objects, an apparatus for simulating the city pollution environment is provided, as defined by the features of claim 1.

In the present invention, an engine is used as the pollution source, which is highly relevant to the city pollution environment, because it is one of the main sources of air pollution in most polluted cities, especially also for pedestrians of such cities walking along urban streets. The engine could be a diesel engine or a gasoline engine.

In the present invention, the sample can be placed in the pollution test chamber and exposed to the pollution environment in three different modes:

1. exhaust gas only;
2. UV irradiation only;
3. exhaust gas and UV irradiation.

The latter combination of UV and exhaust gas has better relevance with regard to simulating the real pollution environment of metropolitan areas and megacities, in which there is both sunshine and exhaust gas present at the same time in the daytime of a polluted city.

**Brief Description of the Drawings**

Figure 1 shows a section view of the city pollution environment simulation apparatus according to the present invention.

Figure 2 shows microscope images of untreated hair fiber surface before and after pollution exposure.

Figure 3 "Exhaust gas component analysis of city pollution chamber" shows results of gas analysis inside the pollution test chamber at different stages. Pollution mode: 0.5h * exhaust gas + 6h UVA.

Figure 4 " PM 2.5 analysis of city pollution chamber" shows results of PM analysis inside the pollution test chamber at different stages. Pollution mode: 0.5h * exhaust gas + 6h UVA.

Figure 5 shows microscope images of treated and untreated hair fiber surface after pollution exposure.

Figure 6 "Comparison of residual combing work of hair of different treatment after pollution exposure" shows comparison of residual combing work results on treated and non-treated hair after pollution exposure. Bleached Chinese hair, n = 3.

Figure 7 "Comparison of Tryptophan of hair of different treatment after pollution exposure" shows comparison of Tryptophan in treated and non-treated hair after pollution exposure. Bleached Chinese hair, n = 3.

Figure 8 "Squalene oxidization products analyzed by GC/MS" shows the results of the squalene oxidization analyzed by GC/MS. Exposure conditions: UVA for 6h; 3 x 30mins diesel engine exhaust gas / Solvent: Ethanol; SQ = Squalene; CSPs = Chain Scission Products; SQOPs = Squalene Oxidation Products; SQ iso-mers = isomers of squalene.

Figure 9 "Sebum oxidization products analyzed by GC/MS" shows the results of the sebum oxidization analyzed by GC/MS. Exposure conditions: UVA for 2h; 1 x 30mins diesel engine exhaust gas. / Solvent: Heptane

Figure 10 "MDA quantification / TBA test results for squalene samples" shows the production of MDA in squalene after pollution exposure. Exposure conditions: UVA for 2h; 1 x 30mins diesel engine exhaust gas / Solvent: Ethanol.

Figure 11 "MDA quantification / TBA test results for sebum samples" shows the production of MDA in sebum after pollution exposure. Exposure conditions: UVA for 2h; 1 x 30mins diesel engine exhaust gas / Solvent: Heptane.

Figure 12 ""Comparison of MDA amount" shows comparison of MDA in sample treated with anti-pollution agent and non-treated sample after pollution exposure. The two samples, on the left side (1) control (sebum only), and on the right side (2) sebum with Moringa oleifera seed extract, after exposure to one cycle in the puv mode in the test pollution chamber

**Mode for Carrying Out the Invention**

The invention described in further details below is based on the objective of providing an apparatus suitable to be used in a laboratory for simulating the city pollution environment in order to evaluate the antipollution efficacy of personal care products on hair, skin or other substrates, with the intention of ensuring that representative and comparative findings can be obtained with regard to the anti-pollution properties of the product.

In order to achieve this objective, an apparatus for simulating the city pollution environment is set up in the laboratory fume hood. The apparatus combines the conditions of air pollution and UV irradiation in the same testing environment. The city pollution mode can be set up with different combinations of UV irradiation and exhaust gas exposure time in order to make the pollution environment situation more flexible for different claims (anti-Particulate Matter, anti-UV, etc.).

The conditions within the pollution test chamber can be monitored and measured with sensors, installed outside the chamber, and connected to the chamber by exhaust gas routing. Such sensors measure the temperature and the humidity, and analyse the gas concentration as well as PM2.5/PM10 particles.

For example, as presented above, a sample can be placed in the chamber and expose to the pollution environment in three different mode:
Either to exhaust gas only or to UV irradiation only or to the combination of exhaust gas and UV irradiation. After defined periods of exposure to combination of different modes, the samples can be taken out for further analysis.

According to one aspect of the present invention, the city pollution simulation apparatus comprises:

- an engine, which could be a diesel engine or a gasoline engine;
- a cooling tank with water circulation, through which an exhaust gas routing is installed, which connects the engine with the pollution test chamber;
- an exhaust gas inlet pipeline, preferably installed at the bottom of the pollution test chamber, to allow the immission of the exhaust gas into the pollution test chamber;
- an exhaust gas outlet pipeline, preferably installed on top of the pollution test chamber, to allow the emission of the exhaust gas out of the pollution test chamber into the fume hood;
- a pollution test chamber,

    - in which UV lights are installed in different positions, preferably on the walls of the pollution test chamber, and wherein,

    - optionally a UV-transparent isolating film is placed between the UV lights and the exhaust gas filling space in order to protect the inner side of the pollution test chamber from contamination from pollution;

    - one or more grid racks installed in the pollution test chamber, preferably horizontally, from which samples can hang (e.g. hair strands) or on which samples can stand (e.g. liquid sample in a beaker or test tube);

    - a perforated plate installed above the inlet of the exhaust gas in the pollution test chamber, preferably on the bottom of the pollution test chamber, in order to distribute the exhaust gas from the engine more homogenously;

    - a temperature sensor and a humidity sensor, which can be combined;

    - an online gas concentration analyzer and a

    - PM2.5/PM10 analyzer system,
    all of which sensors and analyzers are installed outside the pollution test chamber and are connected to chamber with exhaust gas routings in order to monitor the pollution chamber environment.

Preferably the size of the pollution test chamber allows the possibility of the engine and the cooling tank to be installed as well in the laboratory fume hood, meaning the complete city pollution apparatus fits into a conventional laboratory fume hood.

The pollution test chamber of the present invention may be referred to with all kind of different terms, such as "pollution test chamber", as well as "city pollution environment simulation apparatus", "city pollution simulation apparatus" "test pollution chamber", "test chamber"," pollution chamber" or simply "chamber" or "apparatus" in the following.

According to another aspect of the present invention, the city pollution simulation apparatus is operated as follows:
When the engine is turned on and exhaust gas is generated, the exhaust gas is to be cooled before entering the pollution test chamber. Preferably, the exhaust gas enters a pipeline which is immersed in a cooling tank with circulating water, to cool down the hot exhaust gas coming out of the engine to a lower temperature. Preferably the exhaust gas is cooled down to room temperature. After coming out of the cooling tank, the exhaust gas enters the pollution test chamber, preferably from the bottom, first passing through the perforated plate to become more homogenously distributed when entering the main body of the pollution test chamber.

As described further above, in order to conduct the city pollution exposure test, three operation modes are available:

(1) In the case of an exhaust gas only mode, after the test samples are placed in the chamber, the engine is turned on and the exhaust gas will enter the pollution test chamber, fill in the whole chamber space and then dissipate from the outlet pipeline. After a pre-defined period, the engine is turned off and the samples can be taken out for further analysis.

(2) In the case of UV only mode, after the test samples are placed in the chamber, the UV lights are turned on. After a pre-defined period of UV irradiation exposure, the UV lights are switched off and the test samples can be taken out for further analysis.

(3) In the case of UV and exhaust gas mode, after the test samples are placed in the chamber, both the engine and UV lights for example are turned on. After a pre-defined period, the engine and UV lights are turned off and the test samples can be taken out for further analysis.

After a pre-defined period of exhaust gas exposure, e.g. according to case (1) or (3) above, the engine is switched off and the exhaust gas is allowed to flow out of the chamber through a gas outlet pipeline installed on top of the pollution test chamber and to dissipate into the fume hood, without special suction required. Then the samples are taken out for further analysis.

After operation of the city pollution environment simulation apparatus on the UV-irradiation only mode, see e.g. case (2) above, the test samples can be taken out for further analysis immediately after switching off the UV lamps.

The city pollution environment simulation apparatus of present invention can be used in various areas and in different mode of operation, depending on the desired environmental simulation conditions.

The temperature range is not set to specific ranges, and may vary in general from room temperature (around 22 to 25°C) to a temperature of 33 to 36°C. During the operation mode, also UV irradiation makes the air temperature increase within the pollution test chamber. However, in order to avoid observing heat damage as a side effect on samples, the temperature within the pollution test chamber should not be too high (e.g. above 40°C), as this will also not reflect proper average city environmental conditions.

The humidity change within the chamber is dependent on the overall room humidity. At the end of the cycle, the humidity can reach to around 55%-80%, as the exhaust gas brings in water into the chamber when the engine is running.

However, the gas outlet of the pollution test chamber is permanently open during operation, hence there is a continuous heat and humidity exhaust out of the pollution test chamber.

**Methods for testing the Impact and Efficacy of the present Invention**

For example, the city pollution environment simulation apparatus according to the present invention can be used to evaluate damage of light pollution (UV irradiation) and air pollution (exhaust gas) on hair and/or skin before and after applying personal care compositions in order to test their anti-pollution efficacy, e.g. against particulate matter, VOCs, $NO_x$ emission or UV irradiation etc..

The induced hair damages can be investigated using several different evaluation methods:

- A Combing test can help to assess hair aesthetic properties and surface morphology (Residual combing work measurement);
- Differential Scanning Calorimetry (DSC) and biomarker analysis allow the monitoring of chemical changes of hair in composition (e.g. to determine the denaturation temperatures of human hair proteins such as measuring tryptophan degradation or the loss of hair protein);
- Scanning Electron Microscopy (SEM) and bright/dark field microscopy helps to observe aesthetic changes of the hair fibers, particularly the cuticles and pollutant morphology (visualization and imaging).

Using these methods, the anti-pollution efficacy of different individual ingredients and/or formulations (comprising them) can be assessed after exposure of hair strands in the pollution test chamber. These results may indicate if anti-pollution hair care compositions would be effective in a city pollution environment.

**Residual combing work**

Combability can be defined as the subjective perception of the relative ease or difficulty with which human hair can be combed. It depends on the magnitude and on the fluctuations of the forces that oppose combing. Combability is an important attribute, which is always considered when judging the condition of human hair, reflecting the morphological condition of the hair cuticle surface in a macro-scale, i.e. if the cuticle has a lot of lift-up or broken area, there will be an increase of combing force while on the other hand, if the cuticle is well protected and remains in intact status, it should be much easier to comb through the hair and the combing force will be much reduced.

Previously, residual combing work was used mainly for comparing the conditioning performance of hair care products. In operational set-up of the present invention, the method can be applied to evaluate the hair cuticle surface condition before and after pollution exposure in the pollution test chamber in order to understand the damage caused by pollution exposure on the hair morphological and aesthetic properties.

First the hair strands dry combing work is measured before the pollution exposure and then after the pollution exposure experiment.

A standard cleansing surfactant solution may be used to clean the hair strands in order to remove all the pollutants deposited on the hair surface. After drying the hair, the hair strands are to be tested for the dry combing work before and after the pollution exposure. Then the residual combing work is calculated according to below equation:

$$Residual\ combing\ work = \frac{Combing\ work\ after\ pollution}{Combing\ work\ before\ pollution}$$

With increasing pollution there is also continuous increase of residual combing work observed, thereby indicating that the hair cuticle surface become more and more rough and the hair fibers are inclined and entangle more and more during continuous pollution exposure. Although pollutants might be removed away by the cleansing surfactant solution, it is shown that the permanent damage which is caused by pollutants and UV on hair surface persist even after pollutant removal and the cuticle surface is still rough and difficult to comb through.

### Differential scanning calorimetry (DSC)

DSC determines the denaturation temperature of the protein structure of human hair, i.e. the temperature at which the protein structure breaks down upon heating. Human hair consists of alpha-helical (crystalline) proteins embedded in a so-called matrix of amorphous (non-crystalline) protein material. The denaturation temperature is an indicator for the stability of the protein matrix. The lower the temperature, the more the hair is damaged. It is one of the most sensitive methods known for the quantitative determination of internal hair damage. For example, compared to the virgin hair, bleached hair, which has undergone the recognized highly stressful bleaching hair treatment, has a significantly lower Tmax value, thereby indicating the chemical damage caused by hydrogen peroxide during bleaching process. In comparison for hair exposed to pollution, Tmax values may be even lower, indicating that pollution caused hair protein to be more damaged.

### Tryptophan degradation

Tryptophan is an amino acid existing in the human hair keratin. Tryptophan from hair is usually measured directly spectrofluorimetrically as a solid. It is well known that tryptophan is an amino acid sensitive to the UV light. With the increase of pollution exposure, tryptophan continues to decrease as tryptophan degrades under the pollution exposure.

### Hair protein loss measurement

Human hair consists of approximately 80% protein. Proteins are key components in determining the shape of human hairs. In general hair proteins are very stable and can be hardly eluted into water solution. However, under various internal or external stress, e.g. mechanical abrasion, photo irradiation, disease or aging, hair protein tends to elute outside of the hair shaft as a sign of hair damage. Measurement of hair protein elution in solution may be used to understand the hair damage level. Surfactants form a soluble micelle-like complex with proteins causing their dissolution. Compared with water solution, surfactants remove extractable substances from the endocuticle and the cortex, making melanin granules susceptible to removal, moreover, sodium dodecyl sulfate (SDS) causes hair protein denaturation and extracts twice as much protein from hair than water. Hair protein solubilization increases as the pollution increase, showing that the hair becomes more damaged and more fragile when immersed in a surfactant solution and tend to elute more into the solution more.

### Visualization and imaging

Morphological changes in hair surface are undesirable since they cause shine loss, increased roughness and split ends. These effects occur more frequently in the cuticle, which is the outermost layer of the hair strand, and thus the most exposed to the environmental damages (e.g. sunlight irradiation or repeated washing). Particulate matter (PM) deposited on the hair surface can be observed under SEM. Compared with the unpolluted hair, after pollution exposure, there is a significant distribution of particles deposited on the hair surface. The particles deposited on the hair surface are first primer particles with mostly <250 nm of small size, but tend then to agglomerate into big particles of 1-2 micrometers. In SEM photos, the most observed particulates are of less than 0.1 micrometre, which indicates that they derive from the exhaust gas and some of them tend to agglomerate into big particles on the hair surface.

Bright/dark field microscopy may also be used to observe the hair fiber morphological change as well. Compared with the SEM method, bright/dark field microscopy is more convenient to be conducted immediately after a pollution exposure experiment, as it is less costly and may be used to observe hair morphology in a bigger scale for getting a first overview. With increasing of pollution exposure, there are more and more particles deposited on the hair surface and the hair surface look more and more dirty. Even after cleaning, when those deposited particles have been removed, the damaged cuticle surfaces are still detectable. Dark field microscopy can be applied to examine and image the polluted hair strands. Unlike the normal optical microscopy (bright field), in dark field technique, light passing through the specimen is diffracted, reflected, and/or refracted by optical enabling these faint rays to enter the objective. The specimen can then be visualized as a bright object on an otherwise black background. Dark field microscopy may help better identify the deposited particles on the hair surface and the lift-up cuticles edge.

The induced skin damages can be investigated with the dry film method evaluating the pollution effects on skin sebum (and squalene) either

- By Squalene and Sebum oxidation products quantification with GC/MS
  Or
- By Malondialdehyde (MDA) quantification with Spectrophotometer (MDA-TBA detection)

Skin sebum, which is produced by epidermal cells (*keratinocytes*) comprise a mixture of triglycerides (TG) of about 60%, wax esters (WE) of about 28% and squalene of about 12%. Squalene, the latter is a major lipid component of skin surface produced by skin cells, synthesized in the sebaceous glands.

Squalene oxidization may be used as a potential biomarker regarding atmospheric pollution upon skin, because of its disappearance and the formation of peroxidation reactive substances.

## Embodiments of the invention

The pollution test chamber according to the present invention provide the possibility to combine UV lights and exhaust gas in the same space, and to expose samples to defined city pollution conditions. The pollution test chamber can be of any suitable shape, such as square, cylindrical, rectangular, etc. The pollution test chamber can be of any size suitable for a laboratory fume hood, preferably a laboratory bench fume hood. Fume hoods have generally a width from 1000 mm to 2000 mm. The depth may vary between 700 mm and 900 mm, and the height is in general about 2000mm, but can range from 1900 mm up to 2700 mm. Preferably the size of the pollution test chamber is adjusted, that whole city pollution apparatus, including the engine and the cooling tank, can be installed in the fume hood.

One or more UV lights, are preferably installed on the vertical side walls of the pollution test chamber. Optionally, an UV-transparent isolating film, which has a low permeability to liquids, gases, moisture, and organic vapours, but allow full transmittance of ultraviolet radiation is placed between the UV lights and the exhaust gas filling space, to protect the inner wall of the pollution chamber and especially the UV lamps from contamination by the exhaust gas. UV lights can be either UVA lamps or UVB lamps, or combination of both, depending on the desired test settings. However, in order to achieve a homogenous irradiance level, only one type of UV lights should be installed for one experiment, as mixing different types of UV lamps will produce inconsistencies in the light falling on the samples, and may therefore produce inconsistent results.

UVA Lamps are especially suitable for the setting of the present invention, because UVA lamps do not have any UV output below the normal solar cut-off of 295 nm and usually do not degrade materials as fast as UVB lamps. In addition, they usually provide better correlation with actual outdoor weathering.

Thus, preferably, UVA lamps are installed in the pollution chamber, which provide simulation of sunlight in the critical short wavelength region from 365 nm down to the solar cut-off of 295 nm, with a peak emission at 340 nm. These are especially useful for comparison tests of different formulations.

In another embodiment of the invention, alternatively UVB lamps may be installed in the pollution chamber, which provide UVB radiation including the shortest wavelengths of sunlight, which is usually found on the earth's surface. As these UVB lamps emit unnatural, short-wavelengths of UV below the solar cut-off of 295 nm with a peak emission at 313 nm, they can be installed for obtaining faster test results.

One or more grid racks are installed horizontally in the pollution test chamber at any suitable position. On grid racks, samples can either hang, such as those which need to be placed vertically (e.g. hair strands), or can stand such as those samples which need to be placed horizontally (e.g. liquid sample in the beaker or test tube), or a combination of both, so that more samples can be exposed to pollution at the same time and under the same conditions. The grid racks are preferably made of metal, such as stainless steel, steel, alumina.

A perforated plate is installed above the exhaust gas inlet in the pollution chamber, in order to evenly distribute the incoming exhaust gas within the inner space of the pollution test chamber. Preferably the exhaust gas inlet and the perforated plate are installed in the bottom of the chamber.

The samples can be either solid substrates such as hair strands, or artificial skin, or fluid substrates like ready-to-use formulations, or isolated chemical compounds derived from the human body, which can be found on the hair and/or skin surface, like e.g. squalene, synthetic sebum etc., in order to study city pollution effects on these substances independently from their occurrence on the human body. Of course, the hair strands or the artificial skin can be treated with the personal care composition in order to evaluate the protective effect on the treated hair or skin.

However, for personal and environmental safety reasons, the samples evaluated in the pollution test chamber should neither be explosive or inflammable, nor should they have any risk of being harmful for human being and the environment.

**Examples**

E.1 Operation of the pollution test chamber

A city pollution environment simulation apparatus for simulating the city pollution environment was set up and operated in order to evaluate the pollution effect on hair.

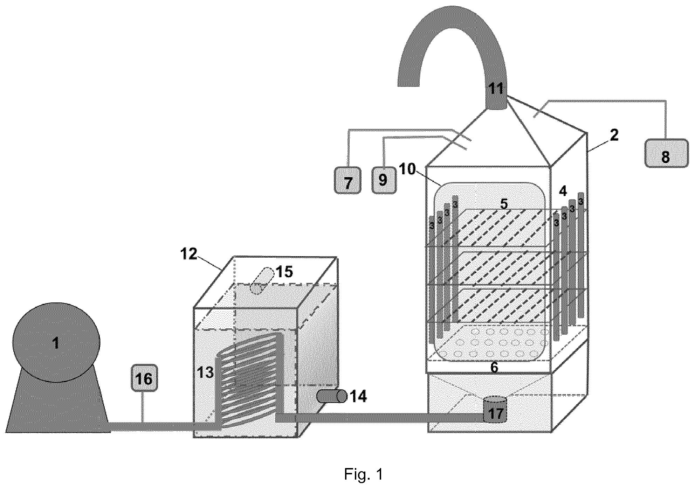

The city pollution environment simulation apparatus according to the present invention and illustrated in Fig. 1 comprises:

a diesel engine (1);
a pollution test chamber (2) which has a width of 40 cm, a length (or depth) of 65 cm and a height of 140 cm, and wherein the chassis of the chamber is made of aluminum alloy having four UVA lights (3) installed on each inner side wall of the pollution test chamber, and wherein an UV-transparent isolating film (4) made of Teflon® FEP is placed between the UV lights and the exhaust gas filling space in order to avoid contamination from exhaust gas;
three rows of hangers (5) made of stainless steel for hair strands are installed horizontally in the middle of the test chamber from the back wall of the chamber to the front in order to let the hair strands to be exposed to full irradiation of UV lights and exhaust gas at the same time;
a perforated plate (6) made of aluminum alloy installed on the bottom of the pollution test chamber above the exhaust gas inlet, in order to distribute the exhaust gas from the engine more homogenously;
three exhaust gas pipelines installed at the top of the pollution test chamber for leading the exhaust gas of the pollution test chamber to the following three sensors:

a temperature/humidity sensor (7);
an online gas concentration analyzer (8); and
a PM2.5/PM10 analyzer system (9)
in order to monitor the pollution chamber environment;
a pollution test chamber door (10) installed in the front of the pollution test chamber;
a gas outlet pipeline (11) installed on top of the pollution test chamber to let go of the exhaust gas into the fume hood;
a gas inlet pipeline (17) installed at the bottom of the pollution test chamber to let the exhaust gas enter into the fume hood;
a cooling tank (12) comprising a water inlet (14) and a water outlet (15) for cooling the exhaust gas, wherein a exhaust gas circuit pipeline (13) is immersed in water, and which pipeline is connected to the engine, and connected to the bottom of the test chamber, a temperature sensor (16) is installed to monitor the temperature of exhaust gas before entering the circuit pipeline.;

When the diesel engine is turned on, exhaust gas is generated. The exhaust gas enters a pipeline which is immersed in circulating water in the cooling tank. After passing the cooling tank, the exhaust gas enters the test chamber from the bottom, first passing through the perforated plate to become more homogenously distributed and then entering the main body of the test chamber.

When the four UVA lights, which are installed on each of the vertical side wall of the pollution chamber, are turned on, they start to emit UV irradiation. The UV irradiation simulates sunlight irradiation in the short wavelength region from 365 nm down to the solar cut-off of 295 nm, with a peak emission at 340 nm.

In this example of operating the city pollution environment simulation apparatus according to the present invention, both the diesel engine and the UV lights are turned on at first.

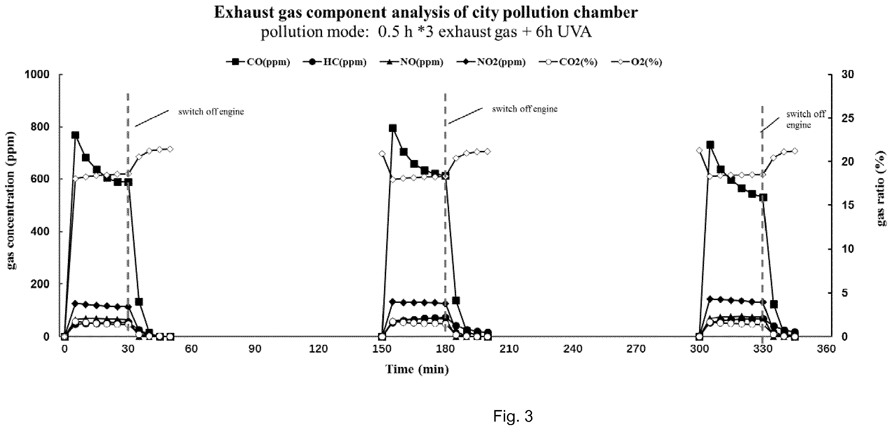

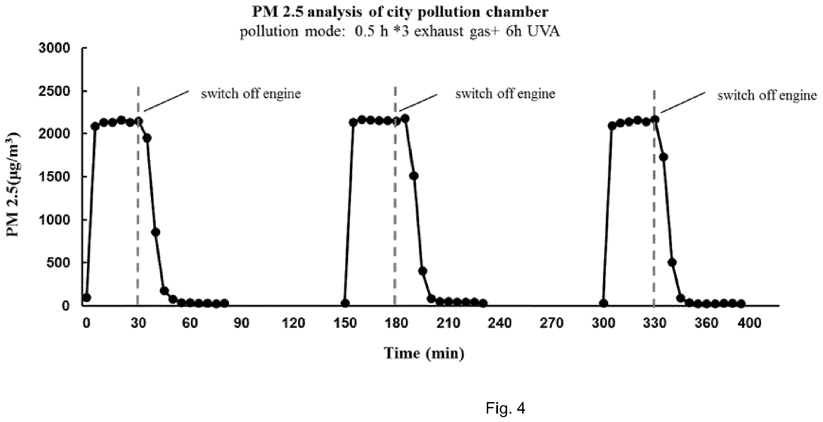

City pollution exposure is simulated as a process of 6h continuous UV irradiation with three periods of diesel engine running, each lasting 30 min. Thus, the diesel engine is turned on at the start at 0h and turned off after 30min, turned on again 2h later at 2.5h from the starting time and then again at 5h during the total 6h period, accordingly, as said above, each time running for 30 minutes. At the end of the testing cycle, the hair strands have been exposed in total to 6 hours of UV irradiation. Although the engine has been generating exhaust gas for 1.5 hours in total, the exposure of the hair strands to exhaust gas is longer than that, as it does not dissipate from the chamber immediately after the engine has been stopped. It can be seen in Figures 3 and 4 showing the gas monitoring graph and the PM analyser graph that even after the engine has been turned off, it takes for another about 30 minutes or even longer for the gas to fully dissipate out of the pollution test chamber.

The results of gas analysis and PM analysis inside the test chamber at different stages are shown in figure 3 and figure 4.

The temperature and the humidity conditions inside the chamber are as below in Table 1.

Table 1. The temperature and the humidity conditions inside the chamber

|  | Beginning of the test (0h) | End of the test (6h) |
|---|---|---|
| Temperature in chamber (°C) | 25.5 | 31.9 |
| Humidity in chamber (%) | 34.5 | 55.4 |

Hair bundles are tied on the hair strands hangers, and are exposed to the pollution environment simulated in the pollution test chamber as described above.

The hair bundles were optically analysed by Microscopy in order to observe the hair surface change before and after exposure to the simulated city pollution conditions.

As shown in figure 2, after exposure to exhaust gas and UVA, it can be seen, that exhaust gas particles deposit on the hair surface and the cuticles lift up.

For evaluation of hair care compositions efficacy, hair strands treated with such hair care compositions would be exposed simultaneously under the same city pollution conditions, and would be analysed before and after exposure.

E.2 Method for evaluating anti-pollution efficacy of hair care composition with pollution test chamber of the present invention

2.1 Test:

Comparison of anti-pollution efficacy of two different polymer solutions and one leave-on formulation on hair strands

2.2 Test objective:

Trial test with hair strands treated with two different polymer solutions and one leave-on formulation, in order to find out the anti-pollution efficacy of the polymer solutions and the leave-on formulation on hair.

2.3 Test preparation

In the trial test, clean bleached Chinese hair strands are used as hair test samples. The tested formulations are of two types, first two basic polymer solutions (table 1) and second one leave-on conditioner containing polymer (table 2).

Table 1. Basic polymer solutions

| Ingredients | Polymer 1 solution I | Polymer 2 solution II |
|---|---|---|
|  | *Concentration in wt %* | *Concentration in wt%* |
| Solvent ($H_2O$) | to 100 | to 100 |
| Polymer 1 (hydrophobically-modified alkali soluble emulsion polymer) [1] | 3 | - |
| Alkalizing agent (NaOH (30%)) | 0.05 | - |
| Polymer 2 (cationic acrylic homopolymer) [2] | - | 1 |

(continued)

| Ingredients | Polymer 1 solution I | Polymer 2 solution II |
|---|---|---|
| | *Concentration in wt %* | *Concentration in wt%* |
| Preservative (DMDM Hydantoin / Iodopropynyl Butylcarbamate) | 0.5 | 0.5 |

*1 Polymer 1 is an associative thickener formulation generally used to enhance the performance of skin and hair care formulations; the final concentration in Polymer 1 Solution I is 1 wt% *2 Polymer 2 is a formulation of a cationic polymer comprising quaternary ammonium functional groups and is generally used for rheology control and showing good spreading properties as well as conditioning properties for hair and skin; the final concentration in Polymer 2 Solution II is 1 wt%

Table 2. Leave-on conditioner formulation

| Ingredients | Leave-on polymer 3 formulation III |
|---|---|
| | *Concentration in wt%* |
| Non-ionic surfactant (Ceteareth-20) | 1 |
| Emulsifier (Cetearyl Alcohol) | 5 |
| Conditioning agent (Coco-Caprylate) | 2 |
| Solvent ($H_2O$) | 86.7 |
| Preservative (DMDM Hydantoin / Iodopropynyl Butylcarbamate) | 0.5 |
| Polymer 3 (cationic VP/DMAEMA copolymer derivative) *3 | 5 |

*3 Polymer 3 is a formulation of cationic polymer comprising quaternary ammonium functional groups and is generally used in hair styling products as well as hair and skin conditioning; the final concentration in Polymer 3 Solution III is 1 wt%

2.3 Test execution:

The hair strands are wetted under the tap water for 1 min and then dried in a climate controlled chamber for 15min (35°C, 50% RH) to a half-dry state. Then the polymer solutions or leave-on formulation are evenly applied on the hair strands (0.3g/2g hair tress), the hair strands are well combed and then leave to dry in the climate controlled room (23°C, 55% RH) overnight. The treated hair are then put in the pollution chamber and exposed to the 6 h pollution cycle (0.5h*3+6h UVA) as described under E.1. Afterwards the hair strands are taken out for cleansing with a standard surfactant solution to remove the deposited pollutants from the hair strands. Then the polymer solutions or leave-on formulation are again evenly applied on the hair strands (0.3g/2g hair tress), the hair strands are well combed and then leave to dry in the climate controlled room (23°C, 55% RH) overnight. The exposure to the 6h pollution cycle is repeated for another 5 times.

| Ingredients | Standard surfactant solution |
|---|---|
| | *Concentration in wt%* |
| Water | 77.9 |
| Cocoyl Amide Propyldimethyl Glycine | 5.4 |
| Sodium Laureth Sulfate | 14.3 |
| Sodium Benzoate | 0.5 |
| Sodium Chloride | 1.9 |

2.4 Test results:

After having been exposed to six 6h pollution cycles in total, the test samples were analyzed. In order to evaluate the antipollution efficacy of the tested formulations, the hair strands were analyzed according the following three evaluation methods (described further above).

2.4.1 Microscopy imaging

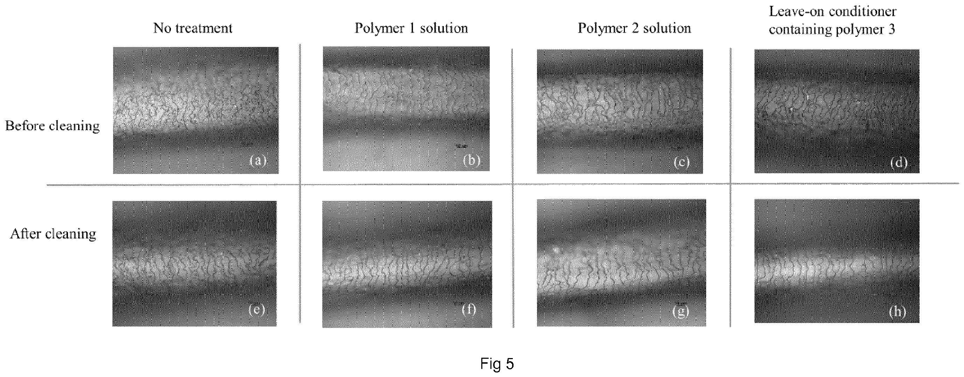

The microscopy photo of polluted hair before and after cleaning were compared. It can be seen, with the application of polymer solution, the cuticle surface of hair looks in better shape than the no-treatment group, with the application of the Leave-on conditioner, the cuticle surface of hair appears even smoother (Figure 5).

2.4.2 Residual combing work test

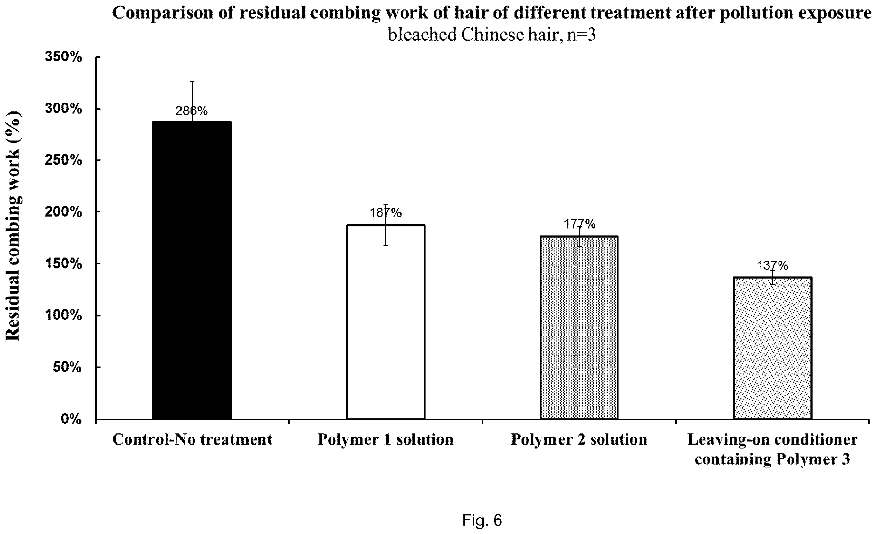

The residual combing work of the three groups of hair strands which have been treated with the three different formulations are significantly much lower than the control group which have not been treated by any formulations and just exposed to the pollution. This indicates that the cuticle surface of the 3 treated groups are much smoother and have lower friction than the control group. As for the 3 group, the two polymer solutions combing work (Polymer 1 solution I and Polymer 2 solution II) are significantly higher than the Leave-on polymer 3 formulation III. The leave-on conditioner contains the cationic polymer in addition to some emollients which all together might boost up the antipollution efficacy of the whole leave-on conditioning formulation (Figure 6).

2.4.3 Tryptophan analysis

For the tryptophan degradation, the group of "No Treatment" is of lowest tryptophan amount, indicating highest level of damage. With the application of polymer 1 and polymer 2 solutions or the Leave-on conditioner with Polymer 3, the tryptophan level increases as well, esp. for the conditioner (Figure 7).

2.5 Test evaluation

In order to simulate the city pollution environment, the city pollution chamber according to the present invention combining both, exhaust gas and UV irradation in the same test environment, showed effective results when evaluating either the effects of city pollution on untreated hair as well as the anti-pollution efficacy of hair care compounds and hair care composition on treated hair.
For the hair exposed in the pollution chamber, various evaluation methods may be utilized to evaluate the hair damage level caused by the pollution.
With the test pollution chamber, it was found that there is a steady increase of combing work of hair after pollution exposure, indicating that the hair cuticle surface become more and more rough due to the pollution exposure. Tryptophan tends to degrade more and hair protein tends to solubilize more into the SDS solution after the pollution exposure. Visualization helps e.g. to identify the morphological change of the hair surface after the pollution exposure which not only shows the damaged cuticle surface but also the pollutants deposited on the hair surface.

E.3 Method for evaluating pollution effects on skin derived compounds with the pollution test chamber of the present invention

3.1 Test:

Evaluation of city pollution effects on skin sebum and squalene with the dry film method.

3.2 Test objective:

As mentioned above, squalene is a major lipid component of skin surface produced by skin cells, synthesized in the sebaceous glands. Its structure $C_{30}H_{50}$, is highly sensitive to oxidization by its six carbon double bonds C=C responsible for its chemical instability. Squalene is soluble in organic solvents (dichloromethane, toluene, benzene etc.).
Sebum, which is produced by epidermal cells (*keratinocytes*) comprise a mixture of triglycerides (TG) of about 60%, wax esters (WE) of about 28% and squalene of about 12%. Squalene oxidization is a potential biomarker regarding atmospheric pollution upon skin, because of its disappearance and the formation of peroxidation reactive substances. These effects observed during exhaust gas exposure and UVA irradiation degradation can be used for evaluating pollution effects (Ekanayake et al., Ultraviolet A Induces Generation of Squalene Monohy-droperoxide Isomers in Human Sebum and Skin Surface Lipids InVitro and InVivo, the Journal of investigative dermatology, Vol. 120, No. 6 June 2003; and K. Jason Dennis, Production of malonaldehyde from squalene, a major skin surface lipid, during UV-irradiation, Photo-chemistry und Photobiology Vol. 49, No. 5, pp. 711-716, 1989)

3.3 Test execution:

A) Stock solutions and preparation:

| Stock solution squalene: | 1 g of squalene was dissolved in 100 ml ethanol solvent |
|---|---|
| Stock solution sebum: | 1 g of synthetic sebum (e.g. Synthetic Sebum 09D, available from WFK Testgewebe GmbH) was dissolved in 100 ml ethanol solvent using a water bath 15 minutes under 40 °C heat |

1 ml of the respective stock solution was added at the center of large petri dishes (100 mm di-ameter x 17 mm height) using an automatic pipette. After evaporating the ethanol solvent in a high ventilated fume hood for 10 mins, the petri dishes were coated with one uniform layer of squalene, respectively sebum, evenly distributed at the bottom of the petri dish.
In total 16 samples were prepared, 8 of each respective batch, meaning 8 with squalene coating (batch 1) and 8 with sebum coating (batch 2).
The side walls of the test petri dish samples were wrapped with plastic band seals in order to get them vertically hooked in a position facing the UVA lamps located on sides in the pollution test chamber.
Four coated petri dishes, two with squalene and two with sebum, were stored in a fridge at 4°C for control purposes, due to the autoxidation of squalene.

B) Exposure of samples in the test pollution chamber:

For each batch, 2 samples (in total 4 samples per mode) were respectively exposed to the 3 different pollution modes (i to iii):

  i. During the "UVA only" ("uv") mode, the samples were exposed to continuous 340 nm UV irradiation of the 8 UVA lamps for 6 hours.
  ii. During the "Pollution only" ("p") mode, samples were exposed, optionally repeatedly, to 30 mins of diesel engine exhaust gas, followed by a 90 min break in between and/or after. Meaning the engine was started and run for 30min, was then switched of for 90 min, and optionally started again afterwards. The samples remained in the test pollution chamber for the full 2 hours, or alternatively for the full 6h, if the engine exhaust gas ran 3 times for 30min.
  iii. During The "Pollution + UV" mode ("puv"), samples were exposed, optionally repeatedly (e.g. 3 times), to the combination of 30 mins diesel engine exhaust gas followed by a 90 min break in between and/or after, and continuous UV irradiation for the full exposure time in the pollution test chamber, which is at least 2 hours, or respectively 6 hours if the engine exhaust gas ran 3 times for 30min with the 90 min break in between and after.

Preparation of samples after exposure for analysis:

After the end of the respective exposure cycle, the "dry film coat" on the bottom of each petri dishes of the 12 exposed and 4 unexposed samples was dissolved by adding 10 ml of organic solvent (ethanol for squalene and heptane for sebum samples) to the petri dish and then transferred into 10 ml vials. Vials were covered with aluminum foil, tightly sealed and covered with parafilm paper to prevent any oxygen-induced reactions and finally stored in the freezer at -20 ° C until analysis.

3.3 Test analysis

3.3.1 Squalene and Sebum oxidation products quantification with GC/MS

Equipment: Agilent 7890B/5977A GC-MS system
Dissolution solvent: Ethanol
column: DB-1MS
Flow Rate: 2ml/min
Column Temperature: 40°C (hold 5min) to 130°C (hold 10 min) by 30°C/min; then to 246°C by 10°C/min; finally, to 300°C (hold 1 min) by 4°C/min.
Inlet: 250°C
Split ratio: 5:1
Inject Volume: 1$\mu$l
Washing solvent: Hexane
MS: range=29 to 700
Solvent delay 3min
MS detection: electrospray ionization ESI

3.3.2. Malondialdehyde (MDA) quantification with Spectrophotometer (MDA-TBA detection)

UV-vis scan between 200 and 800 nm,
Colorimetry: UV detection for MDA-TBA complex at 532nm
Standard curve preparation: As precursor of MDA, 1,1,3,3-tetraethoxypropane (TEP) was used. Stock and Working Solutions: A 10mM TEP stock solution in water was prepared.
Standard solutions, obtained by appropriate dilutions of the above-mentioned stock solution, containing 1 % (v/v) sulfuric acid, were incubated for 2 hours at room temperature to quantitatively release MDA from TEP and to use for calibration purposes.

3.4 Test results

3.4.1 GC/MS

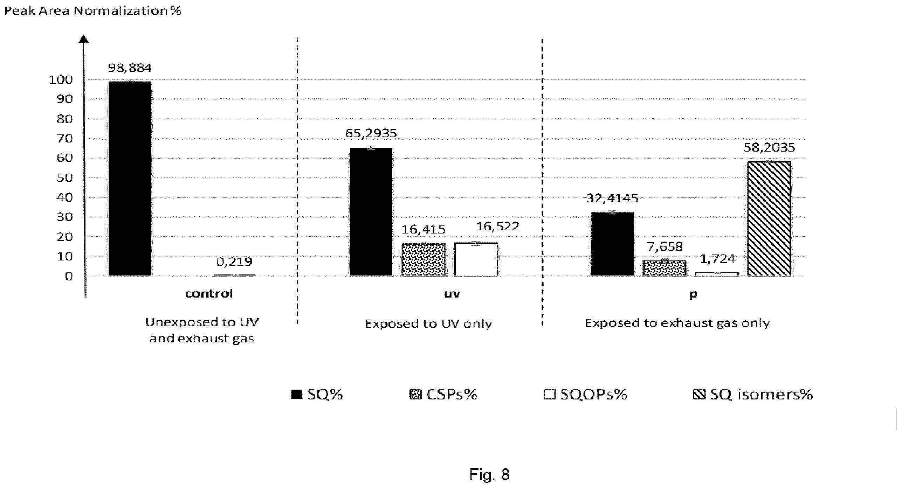

Figure 8 shows the results of the squalene oxidization
Samples, which were exposed to gas pollutants (p-Mode and puv-Mode) lead to squalene isomers (SQ isomers), suggesting an induced isomerization reaction when squalene is in contact with hydrocarbons, nitrogen oxides and carbon oxides.
Samples, which were only exposed to UVA irradiation (UV-Mode) showed the highest content of oxidation products, such as hydroperoxy, hydroxy and epoxy derivatives of squalene having a higher molecular weight higher than squalene (SQOPs).
Samples, which were exposed to UVA and exhaust gas (PUV-Mode) showed a mix of pollution-induced squalene isomers (SQ isomers) as well as irradiation-induced oxidation products (SQOPs).
Among the "chain scission products" (CSPs) of squalene (derived from the scission of hydroperoxy, hydroxy and epoxy derivatives belonging to the SQOP group above), aldehydes, ketones and alcohols could be identified (commonly known compounds include alpha- and beta citral, geranylacetone, farnesal, farnesyl acetaldehyde, geranylgeraniol and 4,9,13,17-tetramethyl-4,8,12,16-octadecatetraenal).

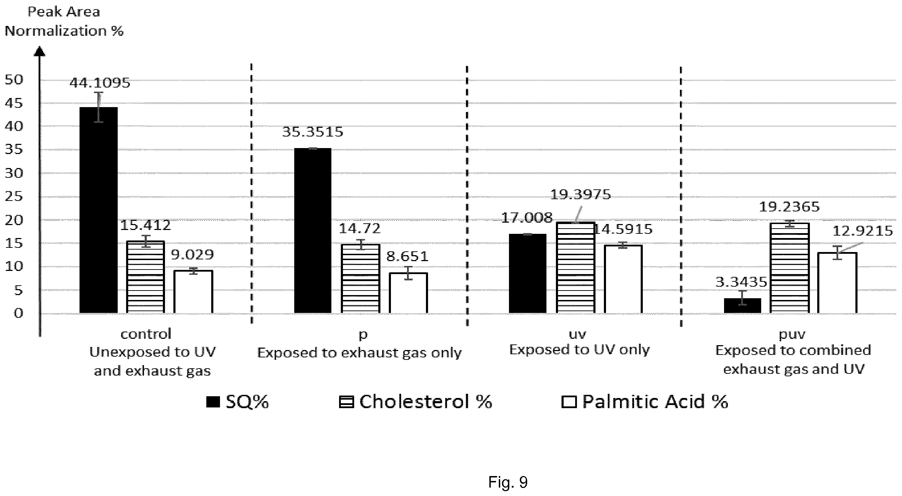

Figure 9 shows the results of the sebum oxidization:
Squalene decrease from sebum was as well observed via GC/MS upon UVA and exhaust gas exposure.
A decrease of 93% of squalene was already detected after 2h of UVA exposure and a one-time 30 min exposure to exhaust gas, whereas cholesterol and palmitic acid, two of the main sebum components appearing in the GC/MS chromatogram showed an increase upon UVA exposure.

3.4.2 Malondialdehyde (MDA) quantification

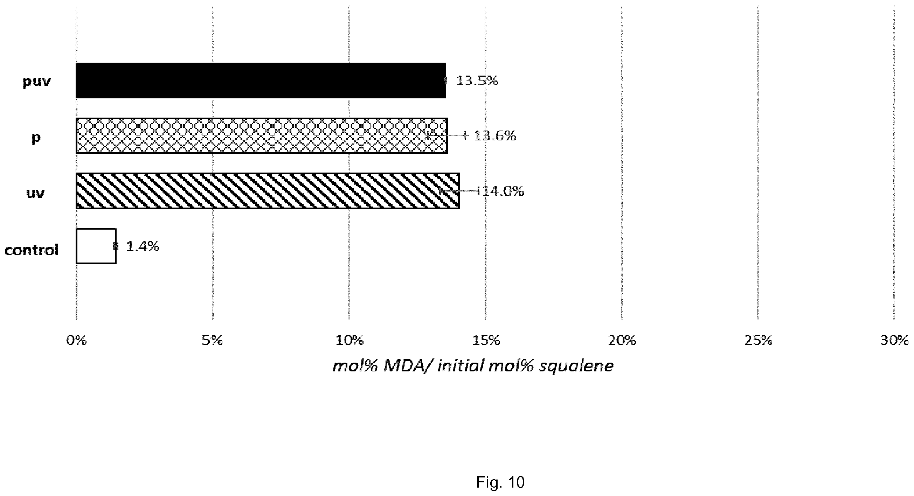

Figure 10 shows correlation between the presence of squalene oxidation products and the production of MDA, lipid peroxidation secondary product.
All samples exposed to the simulated pollution in the pollution test chamber showed a similar significant increase of MDA production compared to the control, no matter which pollution mode was applied. Samples which were exposed to UVA irradiation only ("uv" mode) show a MDA percentage of 14.0% in squalene samples of initial mole of squalene compared to 13.5% and 13.6% of samples respectively exposed only to exhaust gas ("p" mode) or combined exhaust gas and UV irradiation ("puv" mode).

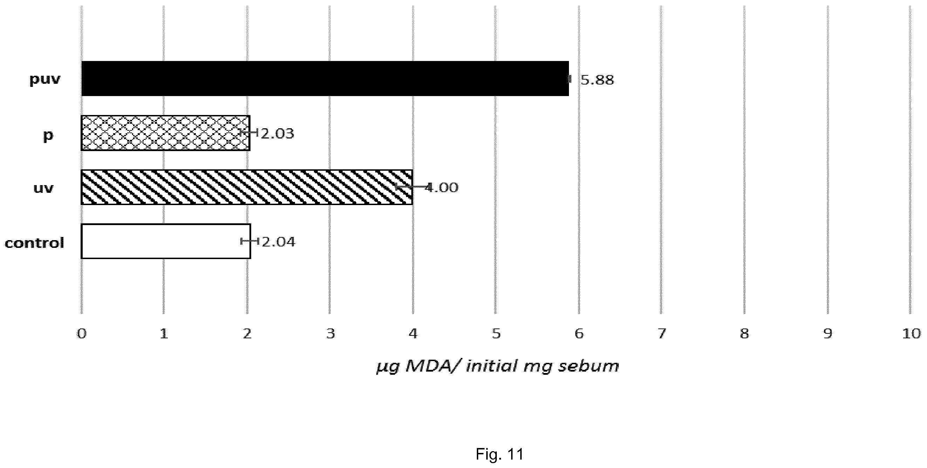

Figure 11 shows correlation between the presence of squalene oxidation products and the production of MDA, lipid peroxidation secondary product.
Samples which were exposed to a combination of UVA and exhaust gas ("puv" mode) showed a higher MDA value (5.88 $\mu$g/mg sebum) compared to samples exposed solely to exhaust gas ("p " mode) (2.03 $\mu$g/mg sebum) or solely to UV irradiation ("uv" mode) (4.00 $\mu$g/mg sebum), thereby suggesting that UVA irradiation in combination with exhaust gas emphasizes sebum oxidization.

E.4 Method for evaluating pollution effects on skin derived compounds and efficacy of anti-pollution agents with the pollution test chamber of the present invention

4.2.1 Test:

Evaluation of in-vitro anti-pollution efficacy against city pollution effects of anti-pollution bio-actives on skin sebum with the dry film method.

4.2.2 Test objective:

As Squalene oxidization is a potential biomarker regarding atmospheric pollution upon skin (see previous test 3.1), said effects can also be used for evaluating the anti-pollution efficacy of anti-pollution agents.

4.2.3 Test execution:

A) Stock solutions and preparation:

Stock solution of Moringa oleifera seed extract:

1.25 wt% aqueous solution of anti-pollution bioactive agent (Purisoft™ PW PSE LS 9836 (Moringa Pterygosperma Seed Extract (and) Maltodextrin))

Stock solution sebum:

1 g of synthetic sebum (e.g. available from WFK Testgewebe GmbH) was dissolved in 100 ml ethanol solvent using a water bath 15 minutes under 40 °C heat

Artificial sebum*

| Ingredient | specified content (by weight) |
|---|---|
| Free fatty acids | 18,0 % |
| Beef tallow | 32,8 % |
| Fattay acid triglycerides | 3,6 % |
| Lanoline | 18,3 % |
| Cholesterol | 3,7 % |
| Hydrocarbon mixture | 12,0 % |
| Cutina | 11,6 % |
| (* available from wfk Testgewebe GmbH) | |

1 ml of the sebum stock solution was added at the center of petri dishes (60 mm di-ameter x 10 mm height) using an automatic pipette. After evaporating the ethanol solvent in the nitrogen working station with constant nitrogen supply for about 1 hour until full evaporation, the petri dishes were coated with one uniform layer of sebum (10 mg sebum in total), evenly distributed at the bottom of the petri dish.

For evaluating the anti-pollution efficacy of the active compound, 1g of the stock solution of Moringa oleifera seed extract were sprayed on the sebum film of the petri dish, forming an aqueous layer containing the anti-pollution compound. After evaporating the water in the nitrogen working station under constant nitrogen supply for 2 hours, the petri dishes were coated with one further uniform film layer of the active compound, evenly distributed on top of the sebum film.

2 samples were prepared, one with sole sebum coating (1) and one with active compound & sebum coating (2). The side walls of the test petri dish samples were wrapped with aluminum foil for protecting light to enter from the side and sealed in order to get them vertically hooked in a position facing the UVA lamps located on sides in the pollution test chamber.

B) Exposure of samples in the test pollution chamber:

Samples were exposed, to the combination of UV radiation and exhaust gas emission. At the beginning of the pollution exposure in the test pollution chamber, UV lights are switched on and the diesel engine is started. After 30 min, the engine is stopped, and the samples remain for another 90 minutes in the text pollution chamber under further UV exposure (1 cycle).

Preparation of samples after exposure for analysis (TBARS method for analyzing MDA):

After pollution exposure, 10 ml heptane was added to the petri dish and then transferred into 10 ml vials. Further, 0.2 ml of a 8 wt% SDS solution and 1.5ml of a 20wt % acetic acid solution was added to each vial and mixed. Afterwards, 1.5 ml of an aqueous 1wt% TBA solution were added to the vial and mixed, followed by the addition of water of up to 4ml in total. The vial was heated to 95 °C for 60minutes, and then cooled down in an ice bath for about 15 minutes. The vial was then kept at room temperature for 15min before being centrifuged at 3000 rpm for 15 min. The solution was measured by UV-Visible Spectrophotometer at expected λAbs of 532nm)
Malondialdehyde (MDA) quantification with Spectrophotometer (MDA-TBA detection by absorbance measurement at expected λAbs of 532nm)
UV-vis scan between 200 and 800 nm,
Colorimetry: UV detection for MDA-TBA complex at 532nm
Standard curve preparation: As precursor of MDA, 1,1,3,3-tetraethoxypropane (TEP) was used.
Stock and Working Solutions: A 10mM TEP stock solution in water was prepared.
Standard solutions, obtained by appropriate dilutions of the above-mentioned stock solution, containing 1 % (v/v) sulfuric acid, were incubated for 2 hours at room temperature to quantitatively release MDA from TEP and to use for calibration purposes.

4.2.4 Test results

Malondialdehyde (MDA) quantification

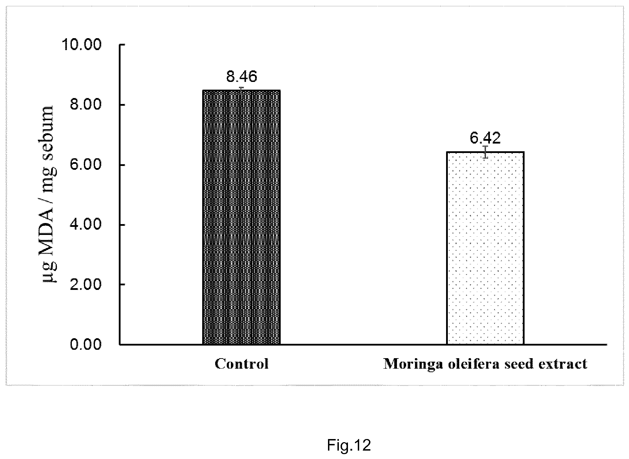

Figure 12 shows the comparison of MDA amount of the two samples, (1) control (sebum only) in column on the left, and (2) sebum with Moringa oleifera seed extract in column on the right, after exposure for 1 cycle in the puv mode in the test pollution chamber. As mentioned before, MDA is the oxidized product of squalene, hence the lower MDA amount in sample (2) indicates antipollution efficacy of the tested compound.

**Claims**

1. A city pollution environment simulation apparatus, comprising an engine (1), a cooling tank (12) and a pollution test chamber (2) comprising one or more UV lights (3), a temperature/humidity sensor (7), an online gas concentration analyzer (8), a PM2.5/PM 10 analyzer system (9), a gas inlet and a gas outlet pipeline (17, 11), **characterized in that** the engine is adapted to emit exhaust gas and is connected via a gas pipeline to the cooling tank, the gas pipeline is in contact with water in the cooling tank, the cooling tank is further connected via the gas pipeline to the pollution test chamber; wherein the one or more UV lights are installed on the walls of the pollution test chamber; wherein the temperature/humidity sensor, the online gas concentration analyzer and the PM2.5/PM10 analyzer system are connected via gas pipelines to the pollution test chamber, and wherein the gas inlet pipeline allows the immission of the exhaust gas into the pollution test chamber and the gas outlet pipeline of the pollution test chamber allows the emission of the exhaust gas out of the pollution test chamber.

2. The city pollution environment simulation apparatus according to claim 1, wherein the gas inlet pipeline (17) is installed at the bottom of the pollution test chamber (2), and the gas outlet pipeline (11) is installed at the top of the pollution test chamber (2).

3. The city pollution environment simulation apparatus according to claim 1 or 2, which comprises a perforated plate (6) above the gas pipeline inlet, preferably at the bottom of the pollution test chamber (2), allowing the exhaust gas passing through the perforated plate and entering the pollution test chamber.

4. The city pollution environment simulation apparatus according to claim 1, 2 or 3, wherein the one or more UV lights (3) are installed on the side wall(s) of the pollution test chamber (2), wherein in particular the city pollution environment simulation apparatus comprises an UV-transparent isolating film (4) placed between the UV lights and the exhaust gas filling space of the pollution test chamber.

5. The city pollution environment simulation apparatus according to any of claims 1-4, wherein the engine (2) is a diesel engine or a gasoline engine.

6. The city pollution environment simulation apparatus according to any of claims 1-5, which comprises one or more grid racks installed in the chamber for hanging samples vertically or supporting samples horizontally, wherein in particular the grid racks are installed from the back wall of the test chamber to the front.

7. The city pollution environment simulation apparatus according to claim 6, wherein the grid racks are metal grid racks, preferably selected from stainless steel, steel or alumina.

8. A method of operating the city pollution environment simulation apparatus of any of claims 1 to 7, wherein the exhaust gas is emitted in the pollution test chamber (2) and the UV lights (3) are emitting UV radiation at the same time.

9. A method of operating the city pollution environment simulation apparatus of any of claims 1 to 7, wherein solely exhaust gas is emitted in the pollution test chamber (2).

10. A method of operating the city pollution environment simulation apparatus of any of claims 1 to 7, wherein solely the UV lights (3) are emitting UV radiation in the pollution test chamber (2).

11. A method according to claim 8, 9 or 10 of operating the city pollution environment simulation apparatus of any of claims 1 to 7for evaluating and testing the effects of engine exhaust gas and/or UV irradiation on samples.

12. A method according to claim 11 of operating the city pollution environment simulation apparatus of any of claims 1 to 7, wherein the methods of claims 8, 9 and 10 are combined in sequence.

13. A method according to claim 11 or 12 of operating the city pollution environment simulation apparatus of any of claims 1 to 7, wherein the samples are hair, artificial skin or non-human derived substrates selected from chemical compounds or formulations, wherein in particular the hair or the artificial skin has been treated with compositions intended to be applied on hair or skin for cosmetic or pharmaceutical purposes.

14. A method for testing anti-pollution efficacy of individual active ingredients, chemical, biological or natural occurring substances, synthesized or derived of natural origin, or cosmetic or pharmaceutical compositions comprising such, by performimg the method according to any of claims 8 to 13 for operating the city pollution environment simulation apparatus of any of claims 1 to 7.

15. A method for testing pollution effects of engine exhaust gas and/or UV radiation on organic material derived from human or non-human origin, such as hair, or on artificial material, e.g. such as artificial skin, or other non-living substrates, by performimg the method according to any of claims 8 to 13 for operating the city pollution environment simulation apparatus of any of claims 1 to 7.

**Patentansprüche**

1. Vorrichtung zur Simulation von städtischer Umweltverschmutzung, die einen Motor (1), einen Kühltank (12) und eine Verschmutzungsprüfkammer (2) umfasst, die ein UV-Licht oder mehrere UV-Lichter (3), einen Temperatur-/Feuchtigkeitssensor (7), einen Online-Gaskonzentrationsanalysator (8), ein PM 2,5/PM 10-Analysesystem (9), eine Gaseinlass- und eine Gasauslassleitung (17, 11) umfasst, **dadurch gekennzeichnet, dass** der Motor so eingerichtet ist, dass er Abgas ausstößt und über eine Gasleitung mit dem Kühltank verbunden ist, die Gasleitung in Kontakt mit Wasser im Kühltank steht, der Kühltank ferner über die Gasleitung mit der Verschmutzungsprüfkammer verbunden ist; wobei das eine UV-Licht oder die mehreren UV-Lichter an den Wänden der Verschmutzungsprüfkammer installiert sind; wobei der Temperatur-/Feuchtigkeitssensor, der Online-Gaskonzentrationsanalysator und das PM 2,5/PM 10-Analysesystem über Gasleitungen mit der Verschmutzungsprüfkammer verbunden sind, und wobei die Gaseinlassleitung die Immission des Abgases in die Verschmutzungsprüfkammer und die Gasauslassleitung der Verschmutzungsprüfkammer die Emission des Abgases aus der Verschmutzungsprüfkammer ermöglicht.

2. Vorrichtung zur Simulation von städtischer Umweltverschmutzung nach Anspruch 1, wobei die Gaseinlassleitung (17) am Boden der Verschmutzungsprüfkammer (2) installiert ist und die Gasauslassleitung (11) am oberen Ende der Verschmutzungsprüfkammer (2) installiert ist.

3. Vorrichtung zur Simulation von städtischer Umweltverschmutzung nach Anspruch 1 oder 2, die eine perforierte Platte (6) oberhalb des Gasleitungseinlasses, bevorzugt am Boden der Verschmutzungsprüfkammer (2), umfasst, die es dem Abgas ermöglicht, durch die perforierte Platte hindurchzugehen und in die Verschmutzungsprüfkammer einzutreten.

4. Vorrichtung zur Simulation von städtischer Umweltverschmutzung nach Anspruch 1, 2 oder 3, wobei das eine UV-Licht oder die mehreren UV-Lichter (3) an der Seitenwand/den Seitenwänden der Verschmutzungsprüfkammer (2) installiert sind, wobei die Vorrichtung zur Simulation von städtischer Umweltverschmutzung insbesondere eine UV-transparente Isolierfolie (4) umfasst, die zwischen den UV-Lichtern und dem Abgasfüllraum der Verschmutzungsprüfkammer angeordnet ist.

5. Vorrichtung zur Simulation von städtischer Umweltverschmutzung nach einem der Ansprüche 1-4, wobei der Motor (2) ein Dieselmotor oder ein Benzinmotor ist.

6. Vorrichtung zur Simulation von städtischer Umweltverschmutzung nach einem der Ansprüche 1-5, die ein oder mehrere in der Kammer installierte Gittergestelle zum vertikalen Aufhängen von Proben oder zum horizontalen Tragen von Proben umfasst, wobei die Gittergestelle insbesondere von der Rückwand der Prüfkammer nach vorne hin installiert sind.

7. Vorrichtung zur Simulation von städtischer Umweltverschmutzung nach Anspruch 6, wobei die Gittergestelle Metallgittergestelle sind, die bevorzugt aus Edelstahl, Stahl oder Aluminiumoxid ausgewählt sind.

8. Verfahren zum Betreiben der Vorrichtung zur Simulation von städtischer Umweltverschmutzung nach einem der Ansprüche 1 bis 7, wobei das Abgas in der Verschmutzungsprüfkammer (2) emittiert wird und die UV-Lichter (3) gleichzeitig UV-Strahlung emittieren.

9. Verfahren zum Betreiben der Vorrichtung zur Simulation von städtischer Umweltverschmutzung nach einem der Ansprüche 1 bis 7, wobei in der Verschmutzungsprüfkammer (2) ausschließlich Abgas emittiert wird.

10. Verfahren zum Betreiben der Vorrichtung zur Simulation von städtischer Umweltverschmutzung nach einem der Ansprüche 1 bis 7, wobei ausschließlich die UV-Lichter (3) in der Verschmutzungsprüfkammer (2) UV-Strahlung emittieren.

11. Verfahren nach Anspruch 8, 9 oder 10 zum Betreiben der Vorrichtung zur Simulation von städtischer Umweltverschmutzung nach einem der Ansprüche 1 bis 7 zum Bewerten und Testen der Auswirkungen von Motorabgas und/oder UV-Bestrahlung auf Proben.

12. Verfahren nach Anspruch 11 zum Betreiben der Vorrichtung zur Simulation von städtischer Umweltverschmutzung nach einem der Ansprüche 1 bis 7, wobei die Verfahren nach den Ansprüchen 8, 9 und 10 der Reihe nach kombiniert werden.

13. Verfahren nach Anspruch 11 oder 12 zum Betreiben der Vorrichtung zur Simulation von städtischer Umweltverschmutzung nach einem der Ansprüche 1 bis 7, wobei die Proben Haare, künstliche Haut oder nicht vom Menschen stammende Substrate sind, die aus chemischen Verbindungen oder Formulierungen ausgewählt sind, wobei insbesondere das Haar oder die künstliche Haut mit Zusammensetzungen behandelt wurde, die dazu bestimmt sind, zu kosmetischen oder pharmazeutischen Zwecken auf das Haar oder die Haut aufgetragen zu werden.

14. Verfahren zum Testen der Anti-Verschmutzungs-Wirksamkeit einzelner Wirkstoffe, chemischer, biologischer oder natürlich vorkommender Substanzen, welche synthetisiert oder von natürlichem Ursprung abgeleitet sind, oder kosmetischer oder pharmazeutischer Zusammensetzungen, die solche enthalten, durch Ausführen des Verfahrens nach einem der Ansprüche 8 bis 13 zum Betreiben der Vorrichtung zur Simulation von städtischer Umweltverschmutzung nach einem der Ansprüche 1 bis 7.

15. Verfahren zum Testen von Verschmutzungseffekten von Motorabgas und/oder UV-Strahlung auf organisches Material menschlichen oder nicht-menschlichen Ursprungs, wie z. B. Haare, oder auf künstliches Material, z. B. wie künstliche Haut, oder andere nicht-lebende Substrate, durch Ausführen des Verfahrens nach einem der Ansprüche 8 bis 13 zum Betreiben der Vorrichtung zur Simulation von städtischer Umweltverschmutzung nach einem der Ansprüche 1 bis 7.

## Revendications

1. Appareil de simulation d'environnement de pollution urbaine, comprenant un moteur (1), une cuve de refroidissement (12) et une chambre d'essai de pollution (2) comprenant une ou plusieurs lampes UV (3), un capteur de température/d'humidité (7), un analyseur de concentration de gaz en ligne (8), un système d'analyse de particules fines PM2.5/PM10 (9), une conduite d'entrée de gaz et de sortie de gaz (17, 11), **caractérisé en ce que** le moteur est adapté pour émettre un gaz d'échappement et est raccordé par le biais d'une conduite de gaz à la cuve de refroidissement, la conduite de gaz est en contact avec de l'eau dans la cuve de refroidissement, la cuve de refroidissement est en outre raccordée par le biais de la conduite de gaz à la chambre d'essai de pollution, dans lequel la ou les lampes UV sont installées sur les parois de la chambre d'essai de pollution, dans lequel le capteur de température/d'humidité, l'analyseur de concentration de gaz en ligne et le système d'analyse de particules fines PM2.5/PM10 sont raccordés par le biais de conduites de gaz à la chambre d'essai de pollution, et dans lequel la conduite d'entrée de gaz permet l'immission du gaz d'échappement à l'intérieur de la chambre d'essai de pollution et la conduite de sortie de gaz de la chambre d'essai de pollution permet l'émission du gaz d'échappement hors de la chambre d'essai de pollution.

2. Appareil de simulation d'environnement de pollution urbaine selon la revendication 1, dans lequel la conduite d'entrée de gaz (17) est installée au fond de la chambre d'essai de pollution (2), et la conduite de sortie de gaz (11) est installée au sommet de la chambre d'essai de pollution (2).

3. Appareil de simulation d'environnement de pollution urbaine selon la revendication 1 ou 2, qui comprend une plaque perforée (6) au-dessus de l'entrée de la conduite de gaz, de préférence au fond de la chambre d'essai de pollution (2), permettant au gaz d'échappement de traverser la plaque perforée et d'entrer dans la chambre d'essai de pollution.

4. Appareil de simulation d'environnement de pollution urbaine selon la revendication 1, 2 ou 3, dans lequel la ou les lampes UV (3) sont installées sur la/les paroi (s) latérale(s) de la chambre d'essai de pollution (2), l'appareil de simulation d'environnement de pollution urbaine comprenant en particulier un film isolant transparent aux UV (4) placé entre les lampes UV et l'espace de remplissage de gaz d'échappement de la chambre d'essai de pollution.

5. Appareil de simulation d'environnement de pollution urbaine selon l'une quelconque des revendications 1 à 4, dans lequel le moteur (2) est un moteur diesel ou un moteur à essence.

6. Appareil de simulation d'environnement de pollution urbaine selon l'une quelconque des revendications 1 à 5, qui comprend une ou plusieurs grilles installées dans la chambre pour suspendre des échantillons verticalement ou supporter des échantillons horizontalement, en particulier dans lequel les grilles sont installées depuis la paroi arrière de la chambre d'essai jusqu'à l'avant.

7. Appareil de simulation d'environnement de pollution urbaine selon la revendication 6, dans lequel les grilles sont des grilles métalliques, de préférence choisies parmi l'acier inoxydable, l'acier et l'alumine.

8. Procédé de fonctionnement de l'appareil de simulation d'environnement de pollution urbaine de l'une quelconque des revendications 1 à 7, dans lequel le gaz d'échappement est émis dans la chambre d'essai de pollution (2) et les lampes UV (3) émettent un rayonnement UV en même temps.

9. Procédé de fonctionnement de l'appareil de simulation d'environnement de pollution urbaine de l'une quelconque des revendications 1 à 7, uniquement dans lequel le gaz d'échappement est émis dans la chambre d'essai de pollution (2).

10. Procédé de fonctionnement de l'appareil de simulation d'environnement de pollution urbaine de l'une quelconque des revendications 1 à 7, uniquement dans lequel les lampes UV (3) émettent un rayonnement UV dans la chambre d'essai de pollution (2).

11. Procédé selon la revendication 8, 9 ou 10 de fonctionnement de l'appareil de simulation d'environnement de pollution urbaine de l'une quelconque des revendications 1 à 7 destiné à évaluer et tester les effets d'un gaz d'échappement de moteur et/ou d'une irradiation UV sur des échantillons.

12. Procédé selon la revendication 11 de fonctionnement de l'appareil de simulation d'environnement de pollution urbaine de l'une quelconque des revendications 1 à 7, dans lequel les procédés des revendications 8, 9 et 10 sont combinés en séquence.

13. Procédé selon la revendication 11 ou 12 de fonctionnement de l'appareil de simulation d'environnement de pollution urbaine de l'une quelconque des revendications 1 à 7, dans lequel les échantillons sont des cheveux, de la peau artificielle ou des substrats d'origine non humaine choisis parmi des composés ou formulations chimiques, en particulier dans lequel les cheveux ou la peau artificielle ont été traités avec des compositions destinées à être appliquées sur les cheveux ou la peau à des fins cosmétiques ou pharmaceutiques.

14. Procédé destiné à tester l'efficacité anti-pollution d'ingrédients actifs individuels, de substances chimiques, biologiques ou présentes dans la nature, synthétisées ou d'origine naturelle, ou de compositions cosmétiques ou pharmaceutiques les comprenant, par réalisation du procédé selon l'une quelconque des revendications 8 à 13 de fonctionnement de l'appareil de simulation d'environnement de pollution urbaine de l'une quelconque des revendications 1 à 7.

15. Procédé destiné à tester les effets de pollution d'un gaz d'échappement de moteur et/ou d'un rayonnement UV sur de la matière organique d'origine humaine ou non humaine, telle que les cheveux, ou sur un matériau artificiel, par ex. de la peau artificielle, ou d'autres substrats non vivants, par réalisation du procédé selon l'une quelconque des revendications 8 à 13 de fonctionnement de l'appareil de simulation d'environnement de pollution urbaine de l'une quelconque des revendications 1 à 7.

Fig. 1

# Before pollution

# After pollution

Fig. 2

Fig. 3

Fig. 4

No treatment | Polymer 1 solution | Polymer 2 solution | Leave-on conditioner containing polymer 3

Before cleaning

(a) (b) (c) (d)

After cleaning

(e) (f) (g) (h)

Fig 5

Comparison of residual combing work of hair of different treatment after pollution exposure
bleached Chinese hair, n=3

Fig. 6

**Comparison of tryptophan of hair of different treatment after pollution exposure**
bleached Chinese hair, n=3

Fig.7

Peak Area Normalization %

Fig. 8

Fig. 9

Fig. 10

Fig. 11

Fig.12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 101887042 A
- KR 101737611 B1
- CN 204583213 U
- CN 104707671 A

**Non-patent literature cited in the description**

- Climate Change and Its Dermatologic Impact on Aging Skin. **HUI Y. et al.** Textbook of Aging Skin. 2015, 1-8
- *EURO COSMETIC,* April 2003, vol. 11, 19
- *Int J Cosmetic Science,* 2015, vol. 37, 357-365
- *Intl Journal of Cosmetic Science,* 2016, 1-4
- **EKANAYAKE et al.** Ultraviolet A Induces Generation of Squalene Monohy-droperoxide Isomers in Human Sebum and Skin Surface Lipids InVitro and InVivo. *Journal of investigative dermatology,* June 2003, vol. 120 (6
- **K. JASON DENNIS.** Production of malonaldehyde from squalene, a major skin surface lipid, during UV-irradiation. *Photo-chemistry und Photobiology,* 1989, vol. 49 (5), 711-716